(19) Europäisches Patentamt
European Patent Office
Office européen des brevets

(11) Veröffentlichungsnummer: **0 252 504**
**B1**

# (12) EUROPÄISCHE PATENTSCHRIFT

(45) Veröffentlichungstag der Patentschrift: **20.06.90**

(51) Int. Cl.⁵: **C07F 9/38**, C07F 9/40, A61K 31/66

(21) Anmeldenummer: **87109899.2**

(22) Anmeldetag: **09.07.87**

## (54) Neue Diphosphonsäurederivate, Verfahren zu deren Herstellung und diese Verbindungen enthaltende Arzneimittel.

(30) Priorität: **11.07.86 DE 3623397**

(43) Veröffentlichungstag der Anmeldung:
**13.01.88 Patentblatt 88/2**

(45) Bekanntmachung des Hinweises auf die Patenterteilung:
**20.06.90 Patentblatt 90/25**

(84) Benannte Vertragsstaaten:
**AT BE CH DE ES FR GB GR IT LI LU NL SE**

(56) Entgegenhaltungen:
**EP-A- 0 175 315**
**DE-A- 2 405 254**
**FR-A- 2 259 615**
**SU-A- 739 076**
**US-A- 3 733 270**

**Fortschritte der Arzneimittelforschung, Band 7, herausgegeben von Ernst Jucker, Basel 1964, Birkhäuser Verlag, Seiten 306-307**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Sandhofer Strasse 116, D-6800 Mannheim 31(DE)**

(72) Erfinder: **Gall, Rudi, Dr. phil., Ulmenstrasse 1, D-6945 Hirschberg 1(DE)**
Erfinder: **Bosies, Elmar, Dr. rer. nat., Delpstrasse 11, D-6940 Weinheim(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 252 504 B1

ACTORUM AG

**Beschreibung**

Die vorliegende Erfindung betrifft neue Diphosphonsäurederivate, Verfahren zu deren Herstellung sowie Arzneimittel, die diese Substanzen enthalten.

In US-A 3 733 270 ist die Verbindung Dizink-2-dibutylamino-1-hydroxyäthan-1-1diphosphonat beschrieben, die zur Verhinderung von Kesselstein Verwendung finden kann.

In der DE-OS 1 813 659 sind Diphosphonsäurederivate beschrieben, von denen die 1-Hydroxy-ethan-1,1-diphosphonsäure als Mittel zur Behandlung von Morbus Paget Bedeutung erlangt hat. In der BE 896 453, EP-A 175 315, DE-A 2 405 254, DE-OS 2 534 391 sowie der EP 96 931 sind Aminoalkan-1,1-diphosphonsäuren als gute Calciumkomplexbildner beschrieben, die sich auch zur Behandlung der erhöhten Knochenresporption einsetzen lassen. Solche Substanzen zeigen bei therapeutisch wirksamer Dosierung häufig Nebenwirkungen. Es stellte sich die Aufgabe, Aminoalkandiphosphonate zu finden, die ihre therapeutische Wirkung bei möglichst niederer Dosierung entfalten. Es wurde nun gefunden, daß analoge Derivate dieser Verbindungen, in denen das Stickstoffatom vollständig alkyliert ist, wobei einer der Alkyl-Reste mindestens 4-C-Atome besitzt, diese Aufgabe erfüllen und als gute Calciumkomplexbildner zur breiteren Behandlung von Calciumstoffwechselstörungen geeignet sind. Sie lassen sich vor allem sehr gut dort einsetzen, wo der Knochenauf- und -abbau gestört ist, d.h. sie sind geeignet zur Behandlung von Erkrankungen des Skelettsystems wie z.B. Osteoporose, Morbus Paget, Morbus Bechterew u.a.

Aufgrund dieser Eigenschaften finden sie aber auch Verwendung in der Therapie von Knochenmetastasen, der Urolithiasis und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenerativen Arthrose.

Gegenstand der vorliegenden Erfindung sind demnach Diphosphonate der allgemeinen Formel I

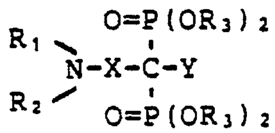

(I),

in der

$R_1$ einen Methyl-, n-Propyl-, Isopropyl-, 3-Methylbutyl-, Pentyl-, Nonyl- oder Cyclohexylmethylrest bedeutet,

$R_2$ eine Butyl-, Isobutyl- 3-Methylbutyl-, Pentyl-, Heptyl-, Nonyl-, Undecyl-, Dodecyl-, Tetradecyl-, Hexadecyl-, Octadecyl-, Cyclohexyl-, Cyclohexylmethyl- oder Benzylgruppe darstellt,

$R_3$ Wasserstoff oder eine Methyl-, Ethyl-, Isopropyl- oder Isobutylgruppe bedeutet,

X eine Methylen-, Ethylen- und Propylengruppe darstellt

und

Y Wasserstoff, Amino oder Hydroxy bedeutet,

sowie deren pharmakologisch unbedenkliche Salze.

Fuer den Rest $R_1$ kommt vorzugsweise der Methyl-, n-Propyl-, Isopropyl-, 3-Methylbutyl-, Pentyl-, und Nonylrest in Frage.

Bevorzugt fuer $R_2$ ist eine Butyl-, Isobutyl-, 3-Methylbutyl-, Pentyl-, Heptyl-, Nonyl-, Decyl-, Undecyl-, Dodecyl-, Tetradecyl-, Hexadecyl-, Octadecyl-, Cyclohexyl-, Cyclohexyl-methyl- oder Benzylgruppe.

Der Rest $R_3$ bedeutet vorzugsweise Wasserstoff oder den Methyl-, Ethyl- oder Isobutylrest.

In $R_1$ , $R_2$ oder X vorkommende asymmettrische Kohlenstoffatome koennen die R-, S- oder R,S-Konfiguration besitzen.

Bevorzugte Verbindungen der vorliegenden Erfindung sind Verbindungen der allgemeinen Formel I, in der $R_1$ ein Methylrest und $R_2$ ein $C_4$-$C_6$-Rest darstellen, insbesondere die Verbindungen 1-Hydroxy-3-(N-methyl-N-pentylamino)-propan-1,1-diphosphonsäure und 1-Hydroxy-3-(N- isobutyl-N-methylamino)-propan-1,1-diphosphonsäure.

Verbindungen der allgemeinen Formel I werden nach an sich bekannten Verfahren hergestellt.

I. Fuer den Fall, daß Y in der allgemeinen Formel I Wasserstoff bedeutet, stellt man die Substanzen vorzugsweise dadurch her, daß man

a) eine Verbindung der allgemeinen Formel II

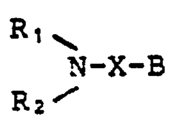

(II),

in der $R_1$ , $R_2$ und X die oben angegebenen Bedeutungen haben und B einen reaktiven Rest wie z. B.

Halogen oder ein Sulfonat darstellt, mit einer Verbindung der allgemeinen Formel III

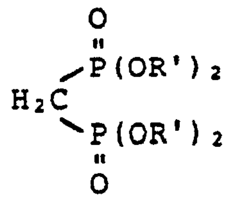 (III),

in der R' fuer Alkylreste mit 1 - 4 Kohlenstoffatomen, vorzugsweise Methyl, Ethyl und Isobutyl steht, zu einem Diphosphonat der allgemeinen Formel IV

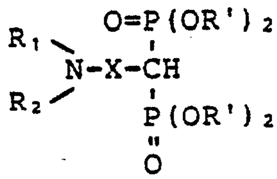 (IV),

in der $R_1$, $R_2$, X und R' die oben angegebene Bedeutung haben, umsetzt und ggf. die entstandenen Tetraester zu Diestern oder Saeuren der allgemeinen Formel I verseift,

oder
b) eine Verbindung der allgemeinen Formel V

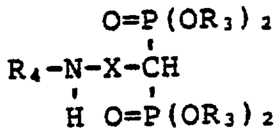 (V),

in der $R_3$ und X die oben angegebene Bedeutung haben und $R_4$ Wasserstoff oder $R_2$ bedeutet, mono- oder dialkyliert,

und ggf. die entstandenen tetraester zu Diestern oder Saeuren der allgemeinen Formel I verseift

oder
II. fuer den Fall, daß Y in der allgemeinen Formel I ggf. durch Alkylgruppen substituiertes Amino bedeutet,

ein Carbonsaeurederivat der allgemeinen Formel VI

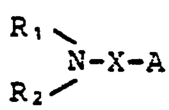 (VI),

in der $R_1$, $R_2$ und X die oben angegebenen Bedeutungen haben und A eine Nitril-, Iminoether- oder eine ggf. am Stickstoff durch niederes Alkyl substituierte Carboxamidogruppe darstellt,

mit einer Phosphorverbindung der allgemeinen Formel VII

$PT_3$(VII),

in der T = Halogen, OH oder OR' bedeutet, wobei R' die oben angegebene Bedeutung hat, umsetzt und ggf. anschließend zu Verbindungen der allgemeinen Formel I verseift,

oder
III. fuer den Fall, daß Y in der allgemeinen Formel I OH bedeutet,
a) eine Carbonsaeure der allgemeinen Formel VIII

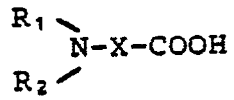

(VIII),

in der $R_1$, $R_2$ und X die oben angegebenen Bedeutungen haben, mit einem Gemisch aus phosphoriger Saeure oder Phosphorsaeure und einem Phosphorhalogenid umsetzt und anschließend zur freien Diphosphonsaeure der allgemeinen Formel I verseift,
oder
b) ein Carbonsaeurechlorid der allgemeinen Formel IX

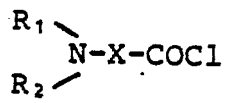

(IX),

in der $R_1$, $R_2$, und X die oben angegebenen Bedeutungen haben, mit einem Trialkylphosphit der allgemeinen Formel X

$P(OR')_3$(X),

in der R' die oben angegebene Bedeutung hat, zu einem Acylphosphonat der allgemeinen Formel XI

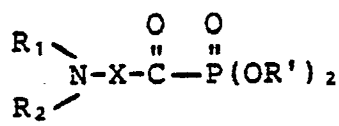

(XI),

in der $R_1$, $R_2$, X und R' die oben angegebenen Bedeutungen haben,
umsetzt, anschließend mit einem Dialkylphosphit der allgemeinen Formel XII

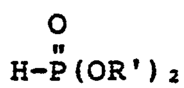

(XII),

in der R' die oben angegebene Bedeutung hat, zu einem Diphosphonat der allgemeinen Formel XIII

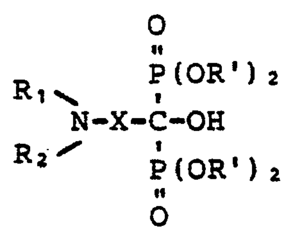

(XIII),

in der $R_1$, $R_2$, X und R' die oben angegebenen Bedeutungen haben, reagieren laeßt und gegebenenfalls die entstandenen Tetraester zu Diestern oder Saeuren der allgemeinen Formel I verseift,

oder
c) eine Verbindung der allgemeinen Formel XIV

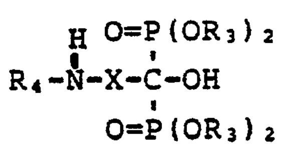

(XIV),

in der $R_3$ und X die oben angegebene Bedeutung haben und $R_4$ Wasserstoff oder $R_2$ bedeutet, mono- oder dialkyliert und gegebenenfalls die entstandenen Tetraester zu Diestern oder Saeuren der allgemeinen Formel I verseift
und die so hergestellten Verbindungen gegebenenfalls in ihre pharmakologisch verträgliche Salze überführt.

Bei Verfahren I a) setzt man den Methylendiphosphonsaeureester der allgemeinen Formel III in Form seines Natrium- oder Kaliumsalzes ein. Hierzu wird er mit Natrium, Kalium oder dem entsrechenden Hydrid in einem inerten Loesungsmittel wie z.B. Benzol, Toluol oder Dimethylformamid bei einer Temperatur von 0 bis 40°C, vorzugsweise bei 25°C umgesetzt. Das Alkalisalz wird ohne Isolierung mit dem entsprechenden Halogenid bzw. Sulfonat zur Reaktion gebracht. Die Temperatur liegt hierbei bei 20 - 110°C.

Bei der reduktiven Alkylierung (Verfahren I b) behandelt man ein Gemisch aus primaerem oder sekundaerem Amin der allgemeinen Formel V und einer Carbonylverbindung oder deren Acetal in Gegenwart eines Hydrierungskatalysators, wie Palladium auf Kohle, oder Nickel, mit Wasserstoff unter Atmosphaeren- oder erhoehtem Druck oder man setzt als reduktionsmittel Ameisensaeure zu. Schließlich lassen sich Alkylierungen eines sekundaeren Amins der allgemeinen Formel V besonders voteilhaft nach dem Phasentransferverfahren mit Dialkylsulfaten durchfuehren.

Bei Verfahren II) setzt man die Nitrile der allgemeinen Formel VI mit phosphoriger Saeure bei Temperaturen von 110 - 180°C um. Die Reaktion kann ohne oder in Gegenwart von aprotischen Loesungsmitteln wie z.B. Diglykoldimethylether oder Diglykoldiethylether durchgefuehrt werden. Man kann die Nitrile jedoch auch mit einem Phosphortrihalogenid, z.B. Phosphortribromid oder Phosphortrichlorid in einem inerten Loesungsmittel wie z.B. Dioxan oder Tetrahydrofuran gegebenenfalls unter Zusatz von Wasser bei Temperaturen von 20 - 80°C zur Reaktion bringen. Iminoether der allgemeinen Formel VI laeßt man mit Dialkylphosphiten vorzugsweise in Gegenwart aequimolarer Mengen Natrium in inerten Loesungsmitteln wie Diethylether, Dioxan oder auch Benzol reagieren, wobei die Umsetzungen in der Regel bei der Rueckflußtempera tur des entsprechenden Loesungsmittels stattfindet. Saeureamide der allgemeinen Formel VI kann man in inerten Loesungsmitteln wie z.B. halogenierten Kohlenwasserstoffen oder Ethern wie z.B. Diethylether mit einem Gemisch aus Phosphorpentahalogenid/phosphoriger Saeure oder auch Oxalylchlorid/Trialkylphosphit umsetzen.

Die bei Verfahren III a) eingesetzten Carbonsaeuren der allgemeinen Formel VIII werden mit 1 - 2, vorzugsweise 1.5 mol phosphoriger Saeure oder Phosphorsaeure und 1 - 2, vorzugsweise 1.5 mol Phosphortrihalogenid bei Temperaturen von 80 - 130°C, vorzugsweise 100 - 110°C umgesetzt. Man kann die Reaktion auch in Gegenwart von Verduennungsmitteln wie Halogenkohlenwasserstoffen, insbesondere Chlorbenzol, Tetrachlorethan oder auch Dioxan durchfuehren. Die anschließende Hydrolyse erfolgt durch Kochen mit Wasser, zweckmaeßigerweise jedoch mit halbkonzentyrierter Salz- oder Bromwasserstoffsaeure.

Bei Verfahren III b) laeßt man das Saeurechlorid der allgemeinen Formel IX mit dem Trialkylphosphit der allgemeinen Formel X bei Temperaturen zwischen 0 und 60°C, vorzugsweise bei 20 - 40°C zur Reaktion kommen. Man kann ohne Loesungsmittel oder auch in Gegenwart von inerten Loesungsmitteln wie Diethylether, Tetrahydrofuran, Dioxan oder auch halogenierten Kohlenwasserstoffen, wie z.B. Methylenchlorid arbeiten. Das als Zwischenprodukt entstehende Acylphosphonat der allgemeinen Formel XI kann isoliert oder direkt weiter umgesetzt werden. Die anschließende Reaktion fuehrt man in Gegenwart einer schwachen Base, vorzugsweise einem sec. Amin wie z.B. Dibutylamin bei einer Temperatur von 0 - 60°C, vorzugsweise bei 10 - 30°C durch.

Als Phosphortrihalogenide kommen in den oben genannten Verfahren beispielsweise Phosphortrichlorid oder Phosphortribromid in Frage.

Bei Verfahren III c) gilt die unter I b gegebene Beschreibung analog.

Die bei Verfahren I und III gegebenenfalls anfallenden Tetraalkylester koennen zu Diestern oder den freien Tetrasaeuren verseift werden. Die Verseifung zu Diestern geschieht in der Regel dadurch, daß man den Tetraalkylester mit einem Alkalihalogenid, vorzugsweise natriumjodid in einem geeigneten Loesungsmittel wie z.B. Aceton bei Zimmertemperatur behandelt.

Hierbei entsteht das symmetrische Diester/Dinatriumsalz, das gegebenenfalls durch einen sauren Ionenaustauscher in die Diester/ Disaeure umgewandelt werden kann. Die Verseifung zu freien Diphosphonsaeuren geschieht in der Regel durch Kochen mit Salz- oder Bromwasserstoffsaeure. Man kann jedoch auch eine Spaltung mit Trimethylsilylhalogenid, vorzugsweise dem Bromid oder Jodid vornehmen. Die freien Diphosphonsaeuren koennen umgekehrt durch Kochen mit Orthoameisensaeurealkylestern wieder in die Tetraalkylester ueberfuehrt werden. Die freien Diphosphonsaeuren der allgemeinen Formel I koennen als freie Saeuren oder in Form ihrer Mono- oder Dialkalisalze isoliert werden. Die Alkalisalze lassen sich in der Regel durch Umfaellen aus Wasser/Methanol oder Wasser/Aceton gut reinigen.

Als pharmakologisch vertraegliche Salze werden vor allem Alkali- oder Ammoniumsalze verwendet, die man in ueblicher Weise z.B. durch Titrieren der Verbindungen mit anorganischen oder organischen Basen wie z.B. Natrium- oder Kaliumhydrogencarbonat, Natronlauge, Kalilauge waessrigem Ammoniak oder Aminen wie z.B. Trimethyl- oder Triethylamin herstellt.

Die erfindungsgemaeßen neuen Substanzen der Formel I und ihre Salze koennen in fluessiger oder fester Form enteral oder parenteral appliziert werden. Hierbei kommen alle ueblichen Applikationsformen in Frage, beispielsweise Tabletten, Kapseln, Dragees, Sirupe, Loesungen, Suspensionen etc.. Als Injektionsmedium kommt vorzugsweise Wasser zur Anwendung, welches die bei Injektionsloesungen ueblichen Zusaetze wie Stabilisierungsmittel, Loesungvermittler und Puffer enthaelt. Derartige Zusaetze sind z.B. Tartrat- und Citrat-Puffer, Ethanol, Komplexbildner (wie Ethylendiamintetraessigsaeure und deren nichttoxische Salze), hochmolekulare Polymere (wie fluessiges Polyethylenoxid) zur Viskositaetsregelung. Fluessige Traegerstoffe fuer Injektionsloesungen muessen steril sein und werden vorzugsweise in Ampullen abgefuellt. Feste Traegerstoffe sind z.B. Staerke, Lactose, Mannit, Methylcellulose, Talkum, hochdisperse Kielselsaeuren, hoehermolekulare Fettsaeuren (wie Stearinsaeure), Gelatine, Agar-Agar, Calciumphosphat, Magnesiumstearat, tierische und pflanzliche Fette, feste hochmolekulare Polymere (wie Polyethylenglykole); fuer orale Applikation geeignete Zubereitungen koennen

gewuenschtenfalls Geschmacks- und Sueßstoffe enthalten.

Die Dosierung kann von verschiedenen Faktoren, wie Applikationsweise, Spezies, Alter und/oder individuellem Zustand abhaengen. Die taeglich zu verabreichenden Dosen liegen bei etwa 1 - 1000 mg/Mensch, vorzugsweise bei 10 - 200 mg/Mensch und koennen auf einmal oder mehrere Male verteilt eingenommen werden.

Bevorzugt im Sinne der vorliegenden Erfindung sind außer den in den Beispielen genannten Verbindungen und den durch Kombination aller in den Anspruechen genannten Bedeutungen ableitbaren Verbindungen die folgenden Diphosphonate sowie deren Methyl- oder Ethylester:

1-Amino-3-(N-methyl-N-nonylamino)propan-1,1-diphosphonsaeure
3-(N-Methyl-N-nonylamino)propan-1,1-diphosphonsaeure
3-(N-Methyl-N-octadecylamino)propan-1-hydroxy-1,1-diphosphonsaeure
3-(N-Methyl-N-tetradecylamino)propan-1-hydroxy-1,1-diphosphonsaeure
3-(N-Heptyl-N-methylamino)propan-1-hydroxy-1,1-diphosphonsaeure
4-(N-Dodecyl-N-methylamino)butan-1-hydroxy-1,1-diphosphonsaeure
3-(N-Dodecyl-N-isopropylamino)propan-1-hydroxy-1,1-diphosphonsauere
1-(Hydroxy-3-(N-cyclohexylmethyl-N-propylamino)propan-1,1-diphosphonsäure
2-(N-Methyl-N-isobutylamino)ethan-1,1-diphosphonsäure
2-(N-Methyl-N-pentylamino)ethan-1,1-diphosphonsäure

Die nachfolgenden Beispiele zeigen einige der Verfahrensvarianten, die zur Synthese der erfindungsgemaeßen Verbindungen verwendet werden koennen. Sie sollen jedoch keine Einschraenkung des Erfindungsgegenstandes darstellen. Die Struktur dieser Verbindungen ist durch H- und P-NMR-Spektroskopie gesichert, die Reinheit mittels P-NMR-Spektroskopie, Duennschichtelektrophorese (Cellulose, Oxalat-Puffer von pH = 4.0) und mittels C,H,N,P,Na-Analyse bestimmt. Zur Charakterisierung der einzelnen Substanzen werden die $M_{rel}$-Werte (= relative Mobilitaet) bezogen auf Pyrophosphat ($M_{rel}$ = 1.0) angegeben.

Beispiel 1

1-Hydroxy-3-(N,N-di-pentylamino)propan-1,1-diphosphonsaeure

13.3. g 3-N,N-Di-pentylamino-propionsaeure werden mit 7.1 g phosphoriger Saeure und 14.8 ml Phosphortrichlorid in 67 ml Chlorbenzol 20 Stunden bei 100°C gehalten. Dann dekantiert man vom Loesungsmittel ab und ruehrt den Rueckstand mit 180 ml 6 N Salzsaeure 8 Stunden unter Rueckfluß. Man filtriert von etwas Unloeslichem ab, konzentriert das Filtrat und gibt es auf eine Amberlite-Saeule IR 120, H±Form. Die Elution mit Wasser wird elektrophoretisch verfolgt. Die gewuenschten Fraktionen werden vereinigt, eingeengt, mit Aceton ausgeruehrt und dei erhaltenen Kristalle isoliert.

Man erhaelt so 12.9 g Rohprodukt. Nach zweimaligem Umkristallisieren aus Wasser erhaelt man 4.7 g = 22 % analysenreines Produkt als Halbhydrat. Fp. 114°C Sintern, 189 - 191°C Zers. ($M_{rel}$: 0.24).

Das Ausgangsmaterial wird wie folgt erhalten:

Dipentylamin wird in Toluol mit Acrylsaeuremethylester im Molverhaeltnis 1:3 umgesetzt. Man erhaelt 28 % des oeligen Dipentyl-aminopropionesters, der mit 1 N Natronlauge verseift wird und 56 % der gewuenschten Saeure liefert, Fp. 47 - 49°C.

Beispiel 2

1-Hydroxy-3-(N-methyl-N-nonylamino)propan-1,1-diphosphonsaeure

In analoger Weise wie in Beispiel 1 beschrieben erhaelt man aus der 3-N-Methyl-N-nonylamino-propionsaeure das entsprechende Diphosphonat mit 10 % Ausbeute, Fp. 159°C Sintern, 178 - 184°C ($M_{rel}$: 0.22).

Das Ausgangsmaterial wird wie folgt erhalten:

Nonylamin wird mit Benzaldehyd in 96 % Ausbeute zur oeligen Schiffschen Base umgesetzt. Die Hydrierung mit Palladium-Kohle-Katalysator gibt mit 94 % Ausbeute N-Benzyl-N-nonylamin, als Oel. Daraus erhaelt man mit Formalin und Ameisensaeure 98 % des oeligen N-Benzyl-N-methyl-N-nonylamins. Die hydrogenolytische Abspaltung des Benzylrestes mit Palladium-Kohle-Katalysator liefert quantitativ das sek. Amin als Oel, das, wie in Beispiel 1 beschrieben, mit Methylacrylat umgesetzt und verseift wird. Ausbeute an Ester 81 % Oel, an Saeure 95 % pastoese Substanz.

Beispiel 3

3-(N-Cyclohexyl-N-methylamino)-1-hydroxy-propan-1,1-diphosphonsaeure

15 g 3-N-Cyclohexyl-N-methylamino-propionsaeure, hergestellt aus N-Cyclohexyl-N-methylamin (Handelspraeparat) und Methylacrylat in Toluol (76 % Ausbeute Ester, Fp. 131 - 134°C; 92 % Ausbeute Saeure, Fp. 101 - 105°C) werden mit 13.3 g phosphoriger Saeure auf 80°C erhitzt. Die Schmelze wird mit 14.1 ml Phosphortrichlorid versetzt und bei gleicher Temperatur 16 Stunden gehalten.

Dann gibt man 240 ml Wasser dazu und ruehrt einen Tag lang bei 100°C. Man filtriert, konzentriert im Vakuum und gießt das Oel in 1 L Aceton, wobei Kristallisation einsetzt. Man loest in Wasser und reinigt durch Ionenaustauscherchromatografie, wie in Beispiel 1 beschrieben. Ausbeute: 4.5 g = 16.9 % Monohydrat. Fp. 142°C Sintern, 182°C Zers. ($M_{rel}$: 0.3)

Beispiel 4

1 g 3-N-Cyclohexylamino-propan-1-hydroxy-1,1-diphosphonsaeure werden in 30 ml Methylenchlorid suspendiert, 2.5 ml konz. Natronlauge zugegeben und unter Kuehlung mit 1 g Tetrabutylammoniumhydrogensulfat und 0.3 ml Dimethylsulfat versetzt. Man ruehrt mehrere Stunden bei Raumtemperatur kraeftig durch. Nach ueblicher Aufarbeitung laeßt sich die Identitaet des erhaltenen produkts mit dem nach Beispiel 3 hergestellten nach Silylierung massenspektrometrisch nachweisen.

Die eingesetzte Diphosphonsaeure wird wie folgt erhalten:

Cyclohexylamin wird in Pyridin mit Acrylsaerue umgesetzt. Ausbeute an 3-N-Cyclohexylamino-propionsaeure 70%, Fp. 170 - 171°C. Die Umsetzung mit phosphoriger Saeure und Phosphortrichlorid liefert 31 % der Diphosphonsaeure vom Fp. 164°C Zers..

Beispiel 5

3-(N-Cyclohexylmethyl-N-methylamino)propan-1-hydroxy-1,1-diphosphonsaeure

3-(N-Cyclohexylmethyl-N-methylamino)propionsaeure (hergestellt aus N-Benzyl-N-methylamin durch Hydrierung mit Platinkatalysator, 70 % Ausbeute, Kp. 60°/16, Umsetzung mit Methylacrylat in Toluol, 37 % Ausbeute an 3-(N-Cyclohexylmethyl-N-methylamino) propionsaeuremethylester, Verseifung mit 1 N Natronlauge zur Saeure, Fp. 98 - 102°C, 63 % Ausbeute). Die Umsetzung mit phosphoriger Saeure/Phosphortrichlorid analog Beispiel 3 liefert 34 % der Diphosphonsaeure, Fp. 180 - 194°C Zers. ($M_{rel}$: 0.31).

Beispiel 6

1-Hydroxy-3-(N-nonyl-N-Propylamino)propan-1,1-diphosphonsaeure

In analoger Weise wie in Beispiel 3 beschrieben, erhaelt man aus der 3-N-Nonyl-N-propylamino-propionsaeure die entsprechende Diphosphonsaeure vom Fp. 100-105°C mit 50 % Ausbeute ($M_{rel}$: 0.23).

Das Ausgangsmaterial wird wie folgt erhalten:

2 mol Nonylamin werden mit 1 mol Propionylchlorid quantitativ zum Saeureamid umgesetzt, das mit Lithiumaluminiumhydrid zum sekundaeren Amin mit 71 % Ausbeute reduziert wird (Kp. 113 - 117°C/16). 1 mol N-Nonyl-N-propylamin werden mit 3 mol Methylacrylat in Toluol umgesetzt und ergeben 81 % eines Oels, das mit 1 N Natronlauge verseift wird und 14 % der gewuenschten Saeure vom Fp. 45 - 47° liefert.

Beispiel 7

500 mg der nach Beispiel 1 hergestellten Diphosphonsaeure werden in 5 ml Wasser suspendiert, mit 2.68 ml 1 N Natronlauge geloest, etwas konzentriert und durch Eingießen in Aceton zur Kristallisation gebracht. Man erhaelt so 440 mg = 78 % des Dinatriumsalzes in Form des Monohydrats der 1-Hydroxy-3-(N,N-dipentyl-amino)propan-1,1-diphosphonsaeure. Der Fp. liegt ueber 300°C.

Beispiel 8

1-Hydroxy-3-(N-nonyl-N-pentylamino)propan-1,1-diphosphonsaeure

2 mol Nonylamin werden mit 1 mol Valeroylchlorid in Ether umgesetzt, die Suspension abgesaugt, das Filtrat eingeengt und so quantitativ das N-Nonyl-verleriansaeureamid vom Fp. 29-31°C erhalten. Die Reduktion mit 1.65 mol Lithiumaluminiumhydrid in Ether gibt 78 % eines farblosen Oels (Kp. 142 - 146°C / 16 Torr). Die Addition dieses N-Nonyl-N-pentylamins an Methylacrylat (96 % Ausbeute, Oel) und nachfolgende Verseifung mit 1 N Natronlauge leifert 64 % einer pastoesen Substanz :

3-(N-Nonyl-N-pentylamino)propionsaeure, die analog Beispiel 3 zur Diphosphonsaeure umgesetzt wird. Ausbeute 87 %, Fp. 168 - 176°C ($M_{rel}$: 0.14).

Beispiel 9

In analoger Weise wie in Beispiel 2 beschrieben werden hergestellt:

| | Ausbeute | Fp. |
|---|---|---|
| A. Zwischenprodukte | | |
| N-Benzyliden-pentylamin | 94% | Oel |
| N-Benzyl-N-pentylamin | 74% | Paste |
| N-Benzyl-N-methyl-N-pentylamin | 95% | Oel |
| N-Methyl-N-pentylamin | 49% | Oel |
| 3-(N-Methyl-N-pentylamino-propionsäure | 93% | Oel |
| 3-(N-Methyl-N-pentylamino-propionsäure | 34% | Zerfliessliche Kristalle |

| Endprodukte: | | |
|---|---|---|
| 1Hydroxy-3-(N-metthyl-N-pentylamino)-propan-1,1-diphosphonsäure $M_{rel}$ = 0,44 | 12% | 84°C Zers. |
| B. Zwischenprodukte | | |
| N-Benzyliden-isobutylamin | 96% | Oel |
| N-Benzyl-N-isobutylamin | 71% | Oel |
| N-Benzyl-N-isobutyl-N-methylamin | 93% | Oel |
| N-Isobutyl-N-methylamin | 96% | Oel |
| 3-(N-Isobutyl-N-methylamino-propionsäure-methyl-ester | 90% | Oel |
| 3-(N-Isobutyl-N-methylamino-propionsäure | 57% | Oel |

| Endprodukte: | | |
|---|---|---|
| 1-Hydroxy-3-(N-isobutyl-N-methylamino)-propan-1,1-diphosphonsäure | | |
| $M_{rel}$ = 0,40 (Monohydrat) | Ausbeute 39% | Fp. 140°C Zers. |

| C. Zwischenprodukte | | |
|---|---|---|
| N-Benzyliden-hexadecylamin | 85% | Oel |
| N-Benzyl-N-hexadecylamin | 71% | Oel |
| N-Benzyl-N-hexadecyl-N-methylamin | 93% | Oel |
| N-Hexadecyl-N-methylamin | 98% | Wachs |
| 3-(N-Hexadecyl-N-methylamino-propionsäuremethylester | 100% | Wachs |
| 3-(N-Hexadecyl-N-methylamino-propionsäure | 37% | 58–60°C |

| Endprodukte: | | |
|---|---|---|
| 3-(N-Hexadecyl-N-methylamino)-propan-1-hydroxy-1,1-diphosphonsäure | | |
| $M_{rel}$ = 0,1 | 72% | 198°/254°C Zers. |

Die öligen Zwischenprodukte werden ohne Destillation direkt weiter umgesetzt. Die Reinigung der Endprodukte erfolgt durch Ionenaustauscherchromatografie.

Beispiel 10

3-N,N-Dinonylaminopropan-1-hydroxy-1,1-diphosphonsäure

In analoger Weise wie in Beispiel 3 beschrieben, erhält man aus der 3-N,N-Dinonylamino-propionsäure die entsprechende Diphosphonsäure als Halbhydrat mit 49% Ausbeute vom Fp. 83°C Sintern, 161-171°C Schmelzen unter Gasentwicklung ($M_{rel}$: 0,16).

Die Reaktionsfolge zur Herstellung des Ausgangsmaterials ist analog der in Beispiel 6 beschriebenen:

Pelargonsäure-N-nonylamid, Ausbeute 100%, Fp. 52 - 55°C;
N,N-Diononylamin, Ausbeute 79%, Fp. 37 - 39°C;
3-N,N-Dinonylamino-propionsäuremethylester, Ausbeute 71%, Oel;
3-N,N-Dinonylamino-propionsäure, Ausbeute 18%, zerfließliche Kristalle.

Beispiel 11

1-Hydroxy-4-(N,N-di-3-methylbutyl-amino)butan-1,1-diphosphonsäure

4 g 4-Amino-1-hydroxybutan-1,1-diphosphonsäure werden in 64 ml 1N Natronlauge gelöst, mit 3,8 ml Isovaleraldehyd versetzt und nach Zugabe von 2,5 g Palladiumkohle 10 % bei 5 Bar hydriert. Der Verlauf wird elektrophoretisch verfolgt, bis das Ausgangsmaterial verschwunden ist. Man filtriert, engt nach Ansäuern mit Amberlite R120-$H^+$-Form ein bis zur Kristallisation und isoliert so 1,3 g Kristalle = 20 % vom Fp. 225 - 227° Zers. ($M_{rel}$ : 0,39). In der Mutterlauge verbliebene, intermediär gebildete 1-Hydroxy-4-(N-3-methylbutyl-amino)butan-1,1-diphosphonsäure kann erneut zur reduktiven Alkylierung eingesetzt werden.

Beispiel 12

3-(N-Benzyl-N-methylamino)propan-1-hydroxy-1,1-diphosphonsäure

Analog Beispiel 3 erhält man aus der 3-N-Benzyl-N-methyl-aminopropionsäure mit 36 % Ausbeute die gewünschte Diphosphonsäure als Monohydrat mit dem Zers.p. 117°C ($M_{rel}$: 0.37).

Das Ausgangsmaterial wird wie folgt erhalten:

N-Benzyl-N-methylamin wird analog Beispiel 1 mit Methylacrylat umgesetzt und der mit 76 % erhaltene ölige Ester ohne Destillation mit 1 N natronlauge verseift. Man erhält so mit 67 % Ausbeute die ölige Säure, die ohne weitere Reinigung eingesetzt wird.

Beispiel 13

3-(N-Dodecyl-N-methylamino)propan-1-hydroxy-1,1-diphosphonsäure

Analog Beispiel 3 erhält man aus der 3-N-Dodecyl-N-methylaminopropionsäure mit 28 % Ausbeute die gewünschte Verbindung. Zers.p. 200-216°C ($M_{rel}$: 0.1).
Das Ausgangsmaterial wird wie folgt erhalten:

Die ölige Schiffsche Base aus Dodecylamin und Benzaldehyd (81 % Ausbeute) wird mit Pd-Katalysator hdyriert: 74 % ölige N-Benzylverbindung. Die reduktive Alkylierung mit Formalin-Ameisensäure gibt mit 82 % ebenfalls öliges tertiäres Amin. Die kat. Abhydrierung des Benzylrestes geht quantitativ. Das ölige sek. Amin wird direkt mit Methylacrylat umgesetzt ( 50 % pastöse Substanz) und ohne Reinigung verseift. Die gewünschte Säure wird mit 39 % als zähe Masse erhalten und direkt eingesetzt.

Beispiel 14

3-(N-Benzyl-N-propylamino)propan-1-hydroxy-1,1-diphosphonsäure

Analog Beispiel 3 erhält man aus der 3-(N-Benzyl-N-propylamino)propionsäure mit 35% Ausbeute die gewünschte Verbindung. Fp. 112-115°C Zers. ($M_{rel}$: 0.33).
Das Ausgangsmaterial wird wie folgt erhalten:

Die ölige Schiff-Base aus Propylamin und Benzaldehyd (86% Ausbeute) wird in Gegenwart von Pd-Katalysator hydriert und gibt mit 81% Ausbeute N-Benzyl-N-propylamin. Das ölige sek. Amin wird nun mit Methylacrylat umgesetzt: 69% öliger Ester und daraus durch alkal. Verseifung die ebenfalls ölige Säure mit 88% Ausbeute erhalten.

Beispiel 15

In analoger Weise wie in Beispiel 2 beschrieben werden hergestellt:

| A. Zwischenprodukte | | |
|---|---|---|
| N-Benzyliden-2-butylamin | 89% | Öl |
| N-Benzyl-2-butylamin | 92% | Öl |
| N-Benzyl-N-2-butyl-N-methylamin | 85% | Öl |
| N-2-Butyl-N-methylamin-HCl | 98 | 40–46°C |
| 3-(N-2-Butyl-N-methylamino)-propionsäuremethylester | 88% | Öl |
| 3-(N-2-Butyl-N-methylamino)-propionsäure | 95% | Öl |

| Endprodukte: | | |
|---|---|---|
| 3-(N-2-Butyl-N-methylamino)-propan-1-hydroxy-1,1-diphosphonsäure | | |
| $M_{rel}$: 0.42 | 39% | 95°–105°C |

| B. Zwischenprodukte | Ausbeute | Fp. |
|---|---|---|
| 3-N-Butylamino-propionsäure-methylester $K_{p20}$: 95–100°C | 75% | Öl |
| 3-(-N-Butyl-N-metylamino)-propionsäure-methylester | – | Öl |
| 3-(N-Butyl-N-methylamino) propionsäure | 78% | Öl |
| (Ausbeute auf 1. Zwischenprodukt bezogen) | | |

| Endprodukte: | | |
|---|---|---|
| 3-(N-Butyl-N-methylamino)-propan-1-hydroxy-1,1-diphosphonsäure | | |
| $M_{rel}$: 0.39 | 65% | 116°–121°C |

| C. Zwischenprodukte | | |
|---|---|---|
| 4-(N-Methyl-N-nonylamino)-buttersäure | 47% | Öl |

| Endprodukte: | | |
|---|---|---|
| 1-Hydroxy-4-(N-methyl-N-nonylamino) butan-1,1-diphosphonsäure-dinatrium-salz-Dihydrat | | |
| $M_{rel}$: 0.25 | 11% | >300°C |

| D. Zwischenprodukte | | |
|---|---|---|
| 3-N-Undecylamino-propionsäure | 62% | 76–80°C |
| 3-N-Methyl-N-undecylamino-propionsäure | 58% | Wachs |

| Endprodukt: | | |
|---|---|---|
| 1-Hydroxy-3-(N-methyl-N-undecylamino) propan-1,1-diphosphonsäure-di-Kaliumsalz-Dihydrat | | |
| | 23% | 238°C Aufsch. |

## Patentansprüche

1. Diphosphonate der allgemeinen Formel I

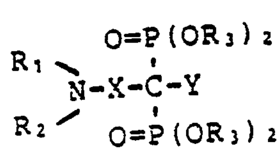

(I),

in der
$R_1$ einen Methyl-, n-Propyl-, Isopropyl-, 3-Methylbutyl-, Pentyl-, Nonyl- oder Cyclohexylmethylrest bedeutet,
$R_2$ eine Butyl-, Isobutyl-, 3-Methylbutyl-, Pentyl-, Heptyl-, Nonyl-, Undecyl-, Dodecyl-, Tetradecyl-, Hexadecyl-, Octadecyl-, Cyclohexyl-, Cyclohexylmethyl- oder Benzylgruppe darstellt,
$R_3$ Wasserstoff oder eine Methyl-, Ethyl-, Isopropyl- oder Isobutylgruppe bedeutet,
X eine Methylen-, Ethylen- und Propylengruppe darstellt und
Y Wasserstoff, Amino oder Hydroxy bedeutet,
sowie deren pharmakologisch unbedenkliche Salze.

2. Diphosphonate der allgemeinen Formel I gemäß Anspruch 1, in der
$R_1$ eine Methyl- oder n-Propylgruppe bedeutet,
$R_2$ eine Isobutyl-, Pentyl-, Nonyl- oder Benzylgruppe darstellt,
$R_3$ Wasserstoff bedeutet,
X eine Ethylengruppe und
Y eine Hydroxygruppe bedeutet,
sowie deren pharmakologisch unbedenkliche Salze.

3. 1-Hydroxy-3-(N-methyl-N-pentylamino)propan-1,1-diphosphonsäure oder 1-Hydroxy-3-(N-isobutyl-N-methylamino)propan-1,1-diphosphonsäure.

4. Verfahren zur Herstellung von Diphosphonaten der allgemeinen Formel I

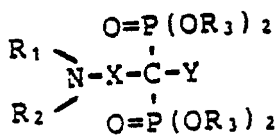 (I),

in der $R_1$ einen Methyl-, n-Propyl-, Isopropyl-, 3-Methylbutyl-, Pentyl-, Nonyl- oder Cyclohexylmethylrest bedeutet,
$R_2$ eine Butyl-, Isobutyl-, 3-Methylbutyl-, Pentyl-, Heptyl-, Nonyl-, Undecyl-, Dodecyl-, Tetradecyl-, Hexadecyl-, Octadecyl-, Cyclohexyl-, Cyclohexylmethyl- oder Benzylgruppe darstellt,
$R_3$ Wasserstoff oder eine Methyl-, Ethyl-, Isopropyl- oder Isobutylgruppe bedeutet,
X eine Methylen-, Ethylen- und Propylengruppe darstellt und
Y Wasserstoff, Amino oder Hydroxy bedeutet,
sowie deren pharmakologisch unbedenkliche Salze, dadurch gekennzeichnet, daß man
I. für den Fall, daß Y in der allgemeinen Formel I Wasserstoff bedeutet,
a) eine Verbindung der allgemeinen Formel II

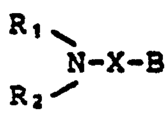 (II),

in der $R_1$, $R_2$ und X die oben angegebenen Bedeutungen haben und B einen reaktiven Rest wie z.B. Halogen oder ein Sulfonat darstellt, mit einer Verbindung der allgemeinen Formel III

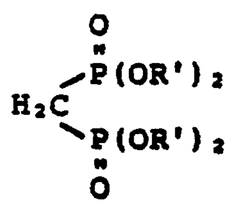 (III),

in der R′ für Alkylreste mit 1–4 Kohlenstoffatomen, vorzugsweise Methyl, Ethyl und Isobutyl steht, zu einem Diphosphonat der allgemeinen Formel IV

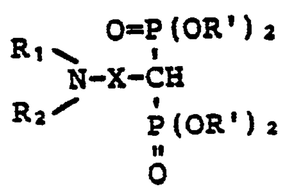 (IV),

in der $R_1$, $R_2$, X und R′ die oben angegebene Bedeutung haben, umsetzt und gegebenenfalls die entstandenen Tetraester zu Diestern oder Säuren der allgemeinen Formel I verseift, oder

b) eine Verbindung der allgemeinen Formel V

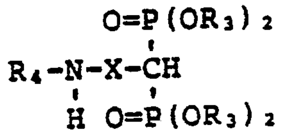 (V),

in der $R_3$ und X die oben angegebene Bedeutung haben und $R_4$ Wasserstoff oder $R_2$ bedeutet, mono- oder dialkyliert,

und gegebenenfalls die entstandenen Tetraester zu Diestern oder Säuren der allgemeinen Formel I verseift oder

II. für den Fall, daß Y in der allgemeinen Formel I Amino, das gegebenenfalls durch Alkylgruppen substituiert ist, bedeutet, ein Carbonsäurederivat der allgemeinen Formel VI

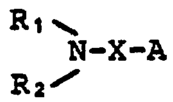 (VI),

in der $R_1$, $R_2$ und X die oben angegebenen Bedeutungen haben und A eine Nitril-, Iminoether- oder eine gegebenenfalls am Stickstoff durch niederes Alkyl substituierte Carboxamidogruppe darstellt, mit einer Phosphorverbindung der allgemeinen Formel VII

$PT_3$ (VII)

in der T = Halogen, OH oder OR′ bedeutet, wobei R′ die oben angegebene Bedeutung hat, umsetzt und gebenenfalls anschließend zu Verbindungen der allgemeinen Formel I verseift oder

III. für den Fall, daß Y in der allgemeinen Formel I OH bedeutet,

a) eine Carbonsäure der allgemeinen Formel VIII

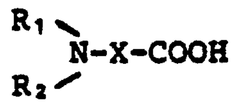 (VIII),

in der $R_1$, $R_2$ und X die oben angegebenen Bedeutungen haben, mit einem Gemisch aus phosphoriger Säure oder Phosphorsäure und einem Phosphorhalogenid umsetzt und anschließend zur freien Diphosphonsäure der allgemeinen Formel I verseift, oder

b) ein Carbonsäurechlorid der allgemeinen Formel IX

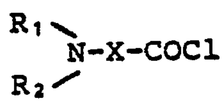 (IX),

in der $R_1$, $R_2$, und X die oben angegebenen Bedeutungen haben, mit einem Trialkylphosphit der allgemeinen Formel X

$P(OR')_3$ (X)

in der R′ die oben angegebene Bedeutung hat, zu einem Acylphosphonat der allgemeinen Formel XI

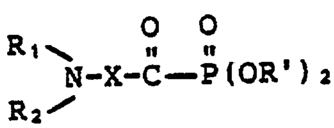 (XI),

in der $R_1$, $R_2$, X und R′ die oben angegebenen Bedeutungen haben, umsetzt, anschließend mit einem Dialkylphosphit der allgemeinen Formel XII

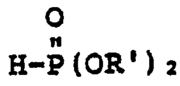 (XII),

in der R′ die oben angegebene Bedeutung hat, zu einem Diphosphonat der allgemeinen Formel XIII

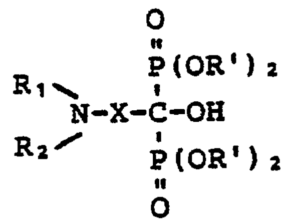 (XIII),

in der $R_1$, $R_2$, X und R′ die oben angegebenen Bedeutungen haben, reagieren läßt und gegebenenfalls die entstandenen Tetraester zu Diestern oder Säuren der allgemeinen Formel I verseift, oder

c) eine Verbindung der allgemeinen Formel XIV

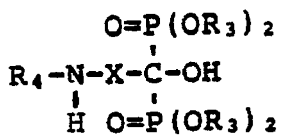 (XIV),

in der $R_3$ und X die oben angegebene Bedeutung haben und $R_4$ Wasserstoff oder $R_2$ bedeutet, mono- oder dialkyliert und gegebenenfalls die entstandenen Tetraester zu Diestern oder Säuren der allgemeinen Formel I verseift und die so hergestellten Verbindungen gegebenenfalls in ihre pharmakologisch verträglichen Salze überführt.

5. Verfahren zur Herstellung von Diphosphonaten der allgemeinen Formel I gemäß Anspruch 4, in der
$R_1$ eine Methyl- oder n-Propylgruppe bedeutet,
$R_2$ eine Isobutyl-, Pentyl-, Nonyl- oder Benzylgruppe darstellt,
$R_3$ Wasserstoff bedeutet,
X ein Ethylengruppe und
Y eine Hydroxygruppe bedeutet,
sowie deren pharmakologisch unbedenkliche Salze.

6. Verfahren gemäß Anspruch 4 zur Herstellung von 1-Hydroxy-3-(N-methyl-N-pentylamino)propan-1,1-diphosphonsäure oder 1-Hydroxy-3-(N-isobutyl-N-methylamino)propan-1,1-diphosponsäure.

7. Arzneimittel enthaltend mindestens eine Verbindung gemäß Anspruch 1, 2 oder 3 sowie übliche pharmakologische Träger- und/oder Hilfsstoffe.

8. Verwendung von Verbindungen gemäß Anspruch 1, 2 oder 3 zur Herstellung von Arzneimitteln.

9. Verwendung von Verbindungen gemäß Anspruch 1, 2 oder 3 zur Herstellung von Arzneimitteln zur Behandlung von Calciumstoffwechselerkrankungen.

**Claims**

1. Diphosphonates of the general formula I

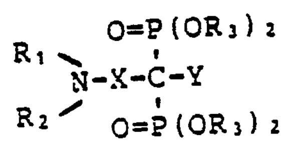 (I),

in which $R_1$ signifies a methyl, n-propyl, isopropyl, 3-methylbutyl, pentyl, nonyl or cyclohexylmethyl radical, $R_2$ represents a butyl, isobutyl, 3-methylbutyl, pentyl, heptyl, nonyl, undecyl, doedecyl, tetradecyl, hexadecyl, octadecyl, cyclohexyl, cyclohexylmethyl or benzyl group, $R_3$ signifies hydrogen or a methyl, ethyl, isopropyl or isobutyl group, X represents a methylene, ethylene and propylene group and Y signifies hydrogen, amino or hydroxyl, as well as their pharmacologically acceptable salts.

2. Diphosphonates of the general formula I according to claim 1, in which $R_1$ signifies a methyl or n-propyl group, $R_2$ represents an isobutyl, pentyl, nonyl or benzyl group, $R_3$ signifies hydrogen, X signifies an ethylene group and Y a hydroxyl group, as well as their pharamcologically acceptable salts.

3. 1-Hydroxy-3-(N-methyl-N-pentylamino)-propane-1,1-diphosphonic acid or 1-hydroxy-3-(N-isobutyl-N-methylamino)-propane-1,1-diphosphonic acid.

4. Process for the preparation of diphosphonates of the general formula I

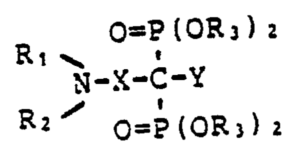 (I),

in which $R_1$ signifies a methyl, n-propyl, isopropyl, 3-methylbutyl, pentyl, nonyl or cyclohexylmethyl radi-

cal, $R_2$ represents a butyl, isobutyl, 3-methylbutyl, pentyl, heptyl, nonyl, undecyl, dodecyl, tetradecyl, hexadeoyl, octadecyl, cyclohexyl, cyclohexylmethyl or benzyl group, $R_3$ signifies hydrogen or a methyl, ethyl, isopropyl or isobutyl group, X represents a methylene, ethylene and propylene group and Y signifies hydrogen, amino or hydroxyl, as well as of their pharmacologically acceptable salts, characterised in that one

I. for the case that Y in general formula I signifies hydrogen,

a) reacts a compound of the general formula II

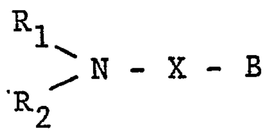

(II)

in which $R_1$, $R_2$ and X have the above-given meanings and B represents a reactive residue, such as e.g. halogen or a sulphonate, with a compound of the general formula III

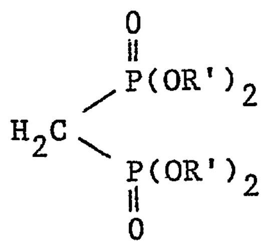

(III)

in which R′ stands for alkyl radicals with 1–4 carbon atoms, preferably methyl, ethyl and isobutyl, to give a disphosphonate of the general formula IV

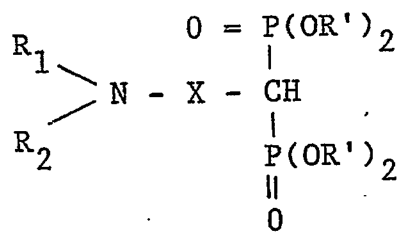

(IV)

in which $R_1$, $R_2$, X and R′ have the above-given meaning, and possibly saponifies the resulting tetraesters to diesters or acids of general formula I, or

b) a compound of the general formula V

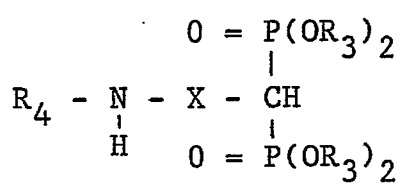

(V)

in which $R_3$ and X have the above-given meaning and $R_4$ signifies hydrogen or $R_2$, and possibly saponifies the resulting tetraesters to diesters or acids of general formula I, or

II. for the case that Y in general formula I signifies amino which is possibly substituted by alkyl groups, reacts a carboxylic acid derivative of the general formula VI

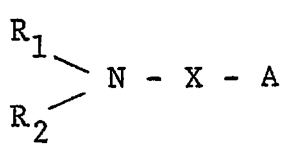

(VI)

in which $R_1$, $R_2$ and X have the above-given meaning and A represents a nitrile, imino ether or a carboxamido group possibly substituted on the nitrogen by lower alkyl, with a phosphorus compound of the general formula VII

$PT_3$ (VII)

in which T = halogen, OH or OR′, whereby R′ has the above-given meaning, and possibly subsequently saponifies to compounds of general formula I, or

III. for the case that Y in general formula I signifies OH
a) reacts a carboxylic acid of the general formula VIII

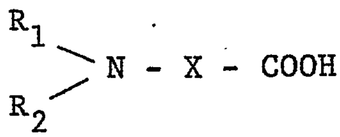 (VIII)

in which $R_1$, $R_2$ and X have the above-given meanings, with a mixture of phosphorous acid or phosphoric acid and a phosphorus halide and subsequently saponifies to the free diphosphonic acid of general formula I, or b) reacts a carboxylic acid chloride of the general formula IX

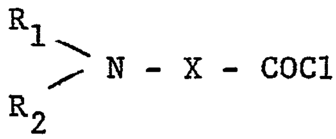 (IX)

in which $R_1$, $R_2$ and X have the above-given meanings, with a trialkyl phosphite of the general formula X
$P(OR')_3$ (X)
in which R′ has the above-given meaning, to give an acyl phosphonate of the general formula XI

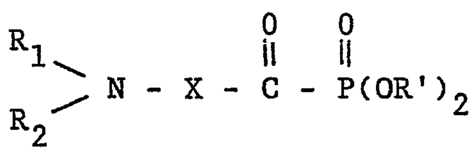 (XI)

in which $R_1$, $R_2$, X and R′ have the above-given meanings, subsequently alows to react with a dialkyl phosphite of the general formula XII

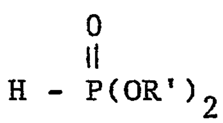 (XII)

in which R′ has the above-given meaning, to give a diphosphonate of the general formula XIII

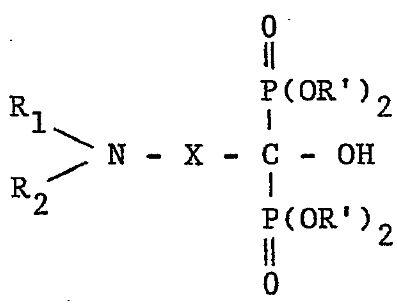 (XIII)

in which $R_1$, $R_2$, X and R′ have the above-given meanings, and possibly saponifies the resulting tetraesters to diesters or acids of general formula I, or
c) a compound of the general formula XIV

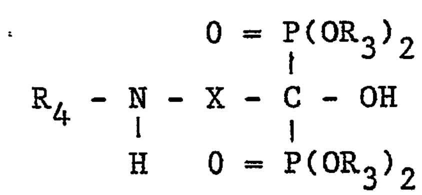 (XIV)

in which $R_3$ and X have the above-given meaning and $R_4$ signifies hydrogen or $R_2$, and possibly saponifies the resulting tetraesters to diesters or acids of general formula I, and possibly converts the so prepared compounds into their pharmacologically acceptable salts.

5. Process for the preparation of diphosphonates of general formula I according to claim 4, in which $R_1$ signifies a methyl or n-propyl group, $R_2$ represents an isobutyl, pentyl, nonyl or benzyl group, $R_3$ sig-

nifies hydrogen, X signifies an ethylene group and Y a hydroxyl group, as well as of their pharmacologically acceptable salts.

6. Process according to claim 4 for the preparation of 1-hydroxy-3-(N-methyl-N-pentylamino)-propane-1,1-diphosphonic acid or of 1–hydroxy-3-(N-isobutyl-N-methylamino)-propane-1,1-diphosphonic acid.

7. Medicaments containing at least one compound according to claim 1, 2 or 3, as well as usual pharmacological carrier and/or adjuvant materials.

8. Use of compounds according to claim 1, 2 or 3 for the preparation of medicaments.

9. Use of compounds according to claim 1, 2 or 3 for the preparation of medicaments for the treatment of calcium metabolism diseases.

**Revendications**

1. Diphosphonates de formule générale I

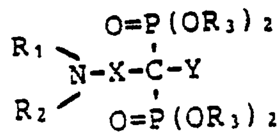
(I),

où

$R_1$ représente un groupe méthyle, n-propyle, isopropyle, 3-méthylbutyle, pentyle, nonyle ou cyclohexylméthyle.

$R_2$ représente un groupe butyle, isobutyle, 3-methylbutyle, pentyle, heptyle, nonyle, undécyle, dodécyle, tétradécyle, hexadécyle, octadécyle, cyclohexyle, cyclohexylméthyle ou benzyle,

$R_3$ représente un atome d'hydrogène, ou un groupe méthyle, éthyle, isopropyle ou isobutyle,

X représente un groupe méthylène, éthylène et propylène, et

Y représente un atome d'hydrogène, un groupe amino ou hydroxy, ainsi que leurs sels pharmaceutiquement acceptables.

2. Diphosphonates de formule générale I selon la revendication 1, où

$R_1$ représente un groupe méthyle ou n-propyle,

$R_2$ représente un groupe isobutyle, pentyle, nonyle, ou benzyle.

$R_3$ represente un atome d'hydrogène,

X représente un groupe éthylène, et

Y représente un groupe hydroxy, ainsi que leurs sels pharmaceutiquement acceptables.

3. Acide 1-hydroxy-3-(N-méthyl-N-pentylamino) propane-1,1-diphosphonique ou acide 1-hydroxy-3-(N-isobutyl-N-méthylamino)propane-1,1-diphosphonique.

4. Procédé de préparation de diphosphonates de formule générale I

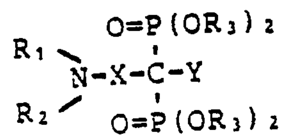
(I),

où

$R_1$ représente un groupe méthyle, n-propyle, isopropyle, 3-méthylbutyle, pentyle, nonyle ou cyclohexylméthyle,

$R_2$ représente un groupe butyle, isobutyle, 3-méthylbutyle, pentyle, heptyle, nonyle, undécyle, dodécyle, tétradécyle, hexadécyle, octadécyle, cyclohexyle, cyclohexylméthyle ou benzyle.

$R_3$ représente un atome d'hydrogène, ou un groupe methyle, ethyle, isopropyle ou isobutyle,

X représente un groupe méthylène, éthylène et propylène, et

Y représente un atome d'hydrogène, un groupe amino ou hydroxy,

ainsi que leurs sels pharamaceutiquement acceptables, caractérisé en ce que

I. dans le cas où Y dans la formule générale I représente l'hydrogène,

a) on fait réagir un composé de formule générale II

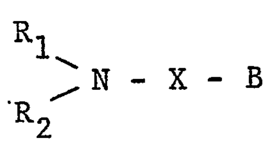
(II)

où $R_1$, $R_2$ et X ont les significations mentionnées ci-dessus, et B représente un groupe réactif comme par exemple un halogène ou un sulfonate, avec un composé de formule générale III

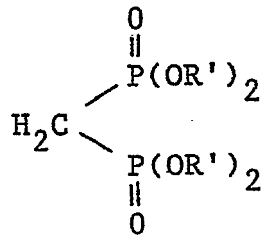

(III)

où R′ représente un groupe alkyle à 1–4 atomes de carbone, de préférence un groupe méthyle, éthyle et isobutyle, en un diphosphonate de formule générale IV

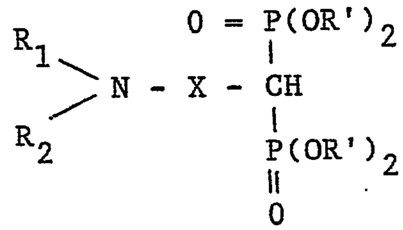

(IV)

où $R_1$, $R_2$, X et R′ ont les significations mentionnées plus haut, et éventuellement on saponifie le tétraester formé en diester ou en acide de formule générale I.

ou b) on soumet un composé de formule générale V

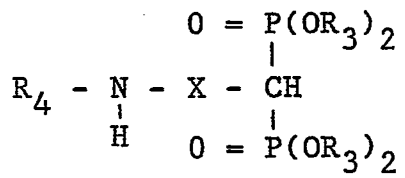

(V)

où $R_3$ et X ont les significations mentionnées plus haut, et $R_4$ représente un atome d'hydrogène ou $R_2$, à une mono-ou dialkylation et eventuellement on saponifie le tétraester formé en diester ou en acide de formule générale I, ou

II. dans le cas où Y dans la formule générale I est un groupe amino, qui est éventuellement substitué par un groupe alkyle, on fait réagir un dérivé d'acide carboxylique de formule générale VI

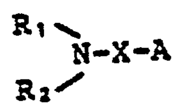

(VI),

où $R_1$, $R_2$ et X ont les significations mentionnées ci-dessus et A représente un groupe nitrile, iminoether ou un groupe carboxyamido éventuellement substitué sur l'azote par un groupe alkyle inférieur, avec un composé du phosphore de formule générale VII

où T = halogène, OH ou OR′, R′ ayant la signification mentionnée plus haut, et ensuite, on saponifie le composé obtenu en composé de formule générale I ou III. dans le cas où Y dans la formule générale I représente un groupe OH, a) on fait réagir un acide carboxylique de formule générale VIII

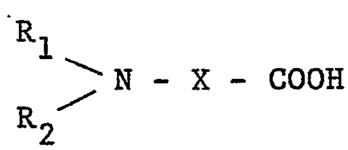

(VIII)

où $R_1$, $R_2$ et X on les significations mentionnées ci-dessus, avec un mélange d'acide phosphoreux ou d'acide phosphorique et d'un halogénure de phosphore, et ensuite, on saponifie en acide diphosphonique libre de formule générale I, ou

b) on fait réagir un chlorure d'acide carboxylique de formule générale IX

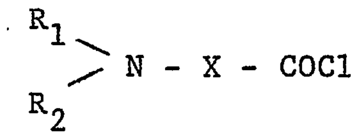 (IX)

où $R_1$, $R_2$ et X ont les significations mentionnées ci-dessus, avec un trialkylphosphite de formule générale X

P(OR')$_3$ (X)

où R' a la signification mentionnée plus haut, en un acylphosphate de formule générale XI

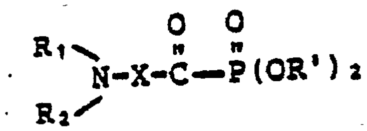 (XI),

où $R_1$, $R_2$, X et R' ont les significations mentionnées plus haut, ensuite, on fait réagir le composé obtenu avec un dialkylphosphite de formule générale XII

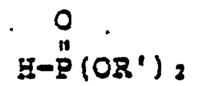 (XII),

où R' à la signification mentionnée plus haut, en un diphosphonate de formule générale XIII

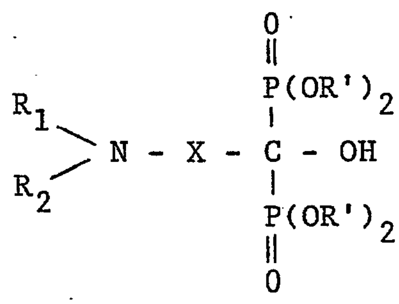 (XIII)

où $R_1$, $R_2$, X et R' ont les significations mentionnées plus haut, et éventuellement on saponifie le tétraester formé en diester ou en acide de formule générale I, ou

c) on soumet un composé de formule générale XIV

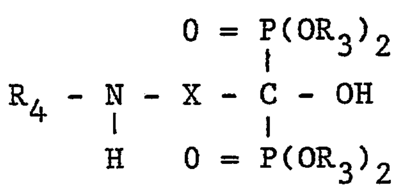 (XIV)

où $R_3$ et X ont la signification mentionnée plus haut, et $R_4$ représente l'hydrogène ou $R_2$, à une mono- ou dialkylation, et éventuellement on saponifie le tétraester formé en diester ou en acide de formule générale I, et on transforme éventuellement le composé ainsi obtenu en un de ses sels pharmaceutiquement acceptables.

5. Procédé de préparation de diphosphonates de formule générale I selon la revendication 4, où
$R_1$ représente un groupe méthyle ou n-propyle,
$R_2$ représente un groupe isobutyle, pentyle, nonyle, ou benzyle,
$R_3$ représente un atome d'hydrogène,
X représente un groupe éthylène, et
Y représente un groupe hydroxy,
ainsi que leurs sels pharmaceutiquement acceptables.

6. Procédé selon la revendication 4, pour la préparation de l'acide 1-hydroxy-3-(N-méthyl-N-pentylamino)propane-1,1-diphosphonique ou de l'acide 1-hydroxy-3-(N-isobutyl-N-méthylamino)-propane-1,1-diphosphonique.

7. Médicament contenant au moins un composé selon les revendications 1, 2 ou 3, ainsi que des véhicules et/ou adjuvants pharmacologiques usuels.

8. Utilisation de composés selon les revendications 1, 2 ou 3, pour la préparation de médicaments.

9. Utilisation de composés selon les revendications 1, 2 ou 3, pour la préparation de médicaments pour le traitement des troubles du métabolisme calcique.